(19) Europäisches Patentamt
European Patent Office
Office européen des brevets

(11) EP 1 022 125 B1

(12) EUROPEAN PATENT SPECIFICATION

(45) Date of publication and mention of the grant of the patent:
**24.11.2004 Bulletin 2004/48**

(51) Int Cl.[7]: **B32B 5/26**, D04H 13/00, A61F 13/15, A41D 31/00, E04D 12/00, B32B 27/12

(21) Application number: **00300471.0**

(22) Date of filing: **21.01.2000**

(54) **Non-woven fabric laminates**

Faservliesverbundmaterialien

Tissus non-tissés laminés

(84) Designated Contracting States:
**DE DK FR**

(30) Priority: **22.01.1999 JP 1490699**
**22.01.1999 JP 1455099**
**02.08.1999 JP 21868799**
**13.10.1999 JP 29112599**

(43) Date of publication of application:
**26.07.2000 Bulletin 2000/30**

(73) Proprietor: **MITSUI CHEMICALS, INC.**
**Tokyo (JP)**

(72) Inventors:
- **Kusano, Kazuya**
**Yokkaichi-shi, Mie (JP)**
- **Sawada, Yuji**
**Ichihara-shi, Chiba (JP)**
- **Motomura, Shigeyuki**
**Yokkaichi-shi, Mie (JP)**
- **Nishino, Kazunari**
**Yokkaichi-shi, Mie (JP)**
- **Nagaoka, Haruki**
**Yokkaichi-shi, Mie (JP)**

(74) Representative: **Cresswell, Thomas Anthony**
**J.A. KEMP & CO.**
**14 South Square**
**Gray's Inn**
**London WC1R 5JJ (GB)**

(56) References cited:

| | |
|---|---|
| EP-A- 0 286 409 | EP-A- 0 309 073 |
| EP-A- 0 611 037 | WO-A-96/16216 |
| WO-A-98/29480 | DE-A- 4 033 633 |
| DE-A- 19 642 252 | US-A- 5 422 172 |
| US-A- 5 514 470 | US-A- 5 597 647 |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 022 125 B1

## Description

TECHNICAL FIELD

The present invention relates to water-vapor-permeable nonwoven fabric laminates. Specifically, it relates to water-vapor-permeable nonwoven fabric laminates which comprise a nonwoven fabric and an elastomer layer having water vapor permeability which are laminated via a layer of an ethylene polymer containing a polar group.

TECHNICAL BACKGROUND

In recent years, nonwoven fabrics whose representative example is spunbonded nonwoven fabric are used for a wide variety of applications. It is demanded that depending on the requirements of individual uses, nonwoven fabrics should be improved in various properties. For example, those nonwoven fabrics used for the gather of disposable diapers, part of sanitary materials such as sanitary napkins, the substrate of compresses, etc. are required to have waterproofness and excellent water vapor permeability. Furthermore, depending on the areas in which such nonwoven fabrics are used, the nonwoven fabrics are required to have excellent stretchability.

For example, sanitary materials such as disposable diapers have such structure that the absorbent material absorbing and holding the body fluid is wrapped by the facing material on the inner side of the absorbent material and the back sheet on the outer side of the absorbent material. The facing material which is in contact with the skin is required to have the function of both allowing the body fluid discharged from the body to permeate through it and be absorbed in the inner absorbent material and preventing the body fluid from flowing back from the absorbent material. On the other hand, the back sheet is require to have both such waterproofness as will not allow the body fluid absorbed in the inner absorbent material to leak to the outside and such appropriate water vapor permeability as will prevent the body from being getting sweaty due to the moisture filled inside and allow the moisture inside of the absorbent product to permeate through it to be dissipated to the outside. Furthermore, since the back sheet constitutes the external surface of the sanitary materials, it is required to have excellent softness and feel.

As examples of those nonwoven fabrics which have excellent softness and feel and show waterproofness and water vapor permeability, the nonwoven fabrics spun by the meltblown method and laminates having strength improved by laminating spunbonded nonwoven fabric thereon can be cited. But these nonwoven fabrics do not have adequate waterproofness when used for such applications as sanitary materials. Because of this, multi-layer sheets are used which comprise nonwoven fabric for the outside surface layer and porous polyethylene film for the inside layer. But the multi-layer sheets used for conventional back sheets involve many manufacturing steps including the step for making polyethylene film porous (e.g. the filled and stretched microporous layer of WO-A-98/29480) and the step for bonding nonwoven fabric and porous polyethylene film by use of a hot-melt adhesive, resulting in a complex manufacturing process and an increase in the cost. Besides, the multi-layer sheets used for conventional back sheets also show poor stretchability.

The nonwoven fabrics now in use for stretchable sheets for the waist part, side panel, side gather, etc. of disposable diapers, among others are stretchable nonwoven fabrics having creases formed by bonding nonwoven fabric to a stretched elastic band. However, this had the problems of not only having poor softness due to stiffness but also involving a complex fabrication process, resulting a higher cost, as a hot-melt adhesive is used in bonding.

As for nonwoven fabrics having stretchability, nonwoven fabrics formed by the melt-blown method using a thermoplastic elastomer composition containing a thermoplastic elastomer or an olefin polymer and nonwoven fabrics made of potentially crimped fiber have been known as such nonwoven fabrics up to the present. The basis weight of the former nonwoven fabrics has to be high, and consequently involves high cost in order to obtain a required level of strength because they have lower strength at break per basis weight than that of those nonwoven fabrics made of general olefin polymer. Moreover, the latter nonwoven fabrics made of potentially crimped fiber develop stretchability as a result of structural changes resulting from the crimping and stretching of the fiber, and their stretchability is limited and only low. As a consequence, these nonwoven fabrics made of potentially crimped fiber have to have higher basis weight and entail higher cost in order to obtain a required level of strength. Besides, both of these two types of nonwoven fabrics show inadequate waterproofness.

SUMMARY

The inventors of the present invention made a serious study to find a solution to the problems with the conventional technology as described above and consequently have reached the present invention.

The invention provides nonwoven fabric laminates having excellent water vapor permeability and waterproofness.

The invention provides nonwoven fabric laminates having excellent interlaminar strength.

The invention provides nonwoven fabric laminates having excellent softness in addition to the above properties.

The invention provides water-vapor-permeable nonwoven fabric laminates which are easy to fabricate.

The invention provides a method for producing water-vapor-permeable nonwoven fabric laminates which does not involve a complex manufacturing process.

The invention provides water-vapor-permeable nonwoven fabric laminates showing water vapor permeability of not less than 1,000 g/$m^2$ · day and water pressure resistance of not less than 1,000 mm Aq, which comprises a layer of a nonwoven fabric(A) made of an olefin polymer and a layer of an elastomer(B) having water vapor permeability which are laminated via a layer of an ethylene polymer(C) containing a polar group, selected from ethylene/ethyl acrylate copolymers, ethylene/vinyl acetate copolymers, ethylene/methacrylic acid copolymers, ionomer resins, modified resins obtainable by modifying ethylene/ethyl acrylate copolymers with unsaturated carboxylic acid and modified resins obtainable by modifying low crystalline copolymers of ethylene and an α-olefin other than ethylene with unsaturated carboxylic acid.

BRIEF EXPLANATION OF THE DRAWINGS

Fig. 1 is a schematic drawing illustrating the extrusion lamination section for obtaining a water-vapor-permeable nonwoven fabric laminate of the invention.

Fig. 2 is a schematic drawing of the cross section of the nonwoven fabric laminate obtained by the extrusion lamination section as shown in Fig. 1.

Fig. 3 is a schematic drawing showing another extrusion lamination section.

Fig. 4 is a schematic drawing of the cross section of the nonwoven fabric laminate obtained by the extrusion lamination section as shown in Fig. 3.

DETAILED DESCRIPTION OF THE INVENTION

The water-vapor-permeable nonwoven fabric laminates of the invention are those water-vapor-permeable nonwoven fabric laminates showing water vapor permeability of not less than 1,000 g/$m^2$ · day and water pressure resistance of not less than 1,000 mm Aq, which comprises a layer of a nonwoven fabric (A) made of an olefin polymer and a layer of an elastomer (B) having water vapor permeability which are laminated via a layer of an ethylene polymer (C) containing a polar group.

Given below is a specific explanation of the nonwoven fabric (A) made of an olefin polymer, the elastomer (B) having water vapor permeability, the ethylene polymer (C) containing a polar group, and the nonwoven fabrics comprising them.

<u>Nonwoven fabric (A) made of olefin polymer</u>:

The nonwoven fabrics of the invention comprise olefin polymer. Examples of the olefin polymer include homopolymers of α-olefins having 2 to 20, preferably 2 to 8, carbon atoms such as ethylene, propylene, 1-butene, 1-pentene, 1-hexene, 4-methyl-1-pentene and 1-octene or copolymers with other α-olefins. Such α-olefins may be selected from among the same olefins as mentioned above.

Specific examples of the olefin polymer include ethylene polymers such as ethylene homopolymer and copolymers of ethylene and another α-olefin such as propylene, 1-butene, 1-pentene, 1-hexene, 4-methyl-1-pentene and 1-octene, propylene polymers such as propylene homopolymer and copolymers of propylene and another α-olefin such as ethylene, polybutene, and poly-4-methyl- 1-pentene.

As ethylene polymers, ethylene homopolymer and copolymer containing not more than 10 mol% or so of another α-olefin are used preferably. Examples of propylene polymers include propylene homopolymer and copolymers of propylene and another α-olefin such as ethylene, 1-butene, 1-hexene, 4-methyl-1-pentene and 1-octene. Such copolymers may be either random copolymers or block copolymers. Out of these, propylene/ethylene random copolymer which comprises propylene and a small amount of ethylene and containing not more than 5 mol% of the structural unit deriving from ethylene is used especially preferably.

These olefin polymers may be used singly or in combination with not less than two of them.

For the method for producing the nonwoven fabric (A) of the invention which is made of olefin polymer, any of the dry method, wet method, spunbonding method and melt-blown method may be used. And from a viewpoint of good productivity and providing the nonwoven (A) having high strength, the spunbonding method is preferable.

In the invention, the embodiment of the nonwoven fabric (A) being a spunbonded fabric is one of the preferable

embodiments of the invention.

Furthermore, in the invention, stretchable nonwoven fabric is preferable as the nonwoven fabric (A). A stretchable nonwoven fabric is a nonwoven fabric whose elongation in either of the longitudinal and transverse directions is not less than 100%, preferably not less than 150%. Elongation of not less than 100% means that the elongation of a nonwoven fabric shown up to the point of failure in tensile test is not less than 100%. As a preferably example of the method for producing such stretchable nonwoven fabric, a method in which a nonwoven fabric produced by the spunbonded method is stretched in the longitudinal direction at temperature lower by 20°C to 40°C than the melting point of the resin may be cited. This enables a high degree of elongation in the transverse direction to be attained. It should be noted that the longitudinal direction means the direction (MD) parallel to the flow of the web at the time of formation of the nonwoven fabric, and the transverse direction means the direction (CD) perpendicular to the flow of the web.

Furthermore, in the invention, the nonwoven fabric used preferably as the nonwoven fabric (A) is a nonwoven fabric made of filaments of the aforesaid olefin polymer and a nonwoven fabric made of conjugate fiber of the core-sheath type or side-by-side type. Conjugate fiber means a fiber is composed of one type of resin and another resin having different properties. Example of such conjugate fiber include core-sheath-type conjugate fiber comprising the core made of one type of resin and the sheath made of another resin and side-by-side-type conjugate fiber composed of two resin parts comprising one resin part and another resin part.

In the invention, an embodiment in which the nonwoven fabric (A) is a core-sheath-type conjugate fiber or a side-by-side-type conjugate fiber is a preferable embodiment of the invention.

Such core-sheath conjugate fiber may be of the concentric type in which the circular core is wrapped in the doughnut-shared sheath having the same center as that of the core or of the eccentric type having a different center from that of the core. Such core-sheath-type conjugate fiber may also be a core-sheath-type conjugate fiber a part of the core of which is exposed to the fiber surface. Out of these, crimped conjugate fiber of the eccentric core-sheath type showing excellent stretchability is preferable.

The resin forming the sheath of such core-sheath-type conjugate fiber may be selected from among the aforesaid olefin polymers as appropriate. Ethylene polymer is preferable from a viewpoint of the softness of the nonwoven fabric. Examples of the ethylene polymers used in the invention include ethylene homopolymer and copolymers of ethylene and $\alpha$-olefin such as propylene, 1-butene, 1-hexene, 4-methyl-1-pentene and 1-octene.

The resin forming the core of such core-sheath-type conjugate fiber may also be selected from among the aforesaid olefin polymers as appropriate, and propylene polymer is preferable. Examples of the propylene polymers used in the invention include propylene homopolymer and copolymers of propylene and $\alpha$-olefin such as ethylene, 1-butene, 1-hexene, 4-methyl-1-pentene and 1-octene. Out of these, propylene/ethylene random copolymer which comprises propylene and a small amount of ethylene and having not more than 5 mol% of the structural unit derived from ethylene. The use of such copolymer allows nonwoven fabric showing satisfactory spinnability and conjugate fiber productivity and have satisfactory softness and excellent feel.

Further, the resin forming the core of the core-sheath-type conjugate fiber is a propylene polymer having the melt flow rate (at 230°; hereinafter referred to as "MFRa") of 0.5 to 100 g/10 minutes, the resin forming the sheath is preferably a different propylene polymer having a different melt flow rate (at 230°; hereinafter referred to as "MFRb") which meets the requirement of the difference in the melt flow rates of the resins of the core and the sheath being MFRa/MFRb $\geqq$ 1.2 or MFRa/MFRb $\leqq$ 0.8. This difference in the melt flow rate makes it easy to obtain crimped conjugate fiber having excellent stretchability. In the invention, Melt flow rate (MFR) was measured at the temperature indicated in each case with the load of 2.16 Kg in accordance with ASTM D1238.

The structural ratio by weight between the resin of the sheath and the resin of the core is preferably in the range of 2/8 to 8/2.

Moreover, the denier of the core-sheath-type conjugate fiber is normally not more than 4 d and preferably not more than 3 d in that a nonwoven fabric having better softness is obtained.

The side-by-side-type conjugate fiber is composed of one type of resin and another type of resin having different properties from those of the former resin. The resins forming the side-by-side-type conjugate fiber are the same resins forming the sheath and core of the aforesaid core-sheath-type conjugate fiber.

The structural ratio by weight between the resin of one part and the resin of the other part of the side-by-side-type conjugate fiber is preferably in the range of 2/8 to 8/2, especially preferably in the range of 3/7 to 7/3 in that this makes it easy to obtain a crimped conjugate fiber.

Moreover, the denier of the side-by-side-type conjugate fiber is normally not more than 4 d and preferably not more than 3 d in that a nonwoven fabric having better softness is obtained.

The nonwoven fabric (A) that is preferably used in the invention is preferably a nonwoven fabric comprising the aforesaid core-sheath-type or side-by-side-type conjugate fiber which is produced by the spunbonding method, for example. Specifically, the resin comprising the core of the core-sheath-type conjugate fiber and the resin comprising its sheath are separately melted in an extruder, etc. and discharged from a spinneret having such conjugate spinning nozzle as is so structured that each melted resin is discharged to form the intended core-sheath structure, so that the

core-sheath-type conjugate fiber is spun. The spun conjugate fiber is cooled in a cooling fluid, given tension by means of stretching air so that the fiber is of a specified denier, and collected and stacked on the collecting belt to specified thickness so that a web of conjugate fiber is obtained. This enables high-strength nonwoven fabric to be obtained and stretchable crimpted conjugate fiber of the eccesntric core-sheath-type to be produced.

After that, nonwoven fabric is fabricated by getting the fiber entangled by the heat embossing processing using embossing rolls. The embossing area ratio (stamping area ratio: ratio of the heat bonded area to the entire nonwoven fabric area) in the heat embossing processing may be determined as appropriate to meet the requirements of individual applications. It is normally possible to obtain conjugate fiber nonwoven fabric having a good balance among solftness, gas permeability and abrasion resistance by causing the embossing area ratio to be in the range of 5% to 40%.

The use of a nozzle for spinning side-by-side-type conjugate fiber, in place of the aforesaid nozzle for spinning core-sheath-type conjugate fiber, allows nonwoven fabric made of the side-by-side-type conjugate fiber of the invention to be obtained. This enables high-strength nonwoven fabric and stretchable crimped conjugate fiber to be produced.

In the invention, an embodiment of the nonwoven fabric (A) being crimped conjugate fiber is also a preferable embodiment of the invention.

Those nonwoven fabrics of the invention which have the basis weight of not more than 30 $g/m^2$ are normally suitable for use for those applications requiring softness. But those nonwoven fabrics having a high basis weight of more than 30 $g/m^2$ may be used for some applications.

<u>Elastomer (B) having water vapor permeability</u>:

The material used for the formation of the film to be laminated to the nonwoven fabrics of the invention is the elastomer (B) having water vapor permeability. The aforesaid elastomer is an elastic high-molecular weight material. A preferable example of the elastomer is thermoplastic elastomer from the viewpoint of moldability. Such thermoplastic elastomer is a high-molecular weight material that has elastic properties similar to those of vulcanized rubber (because of the soft segment in the molecules) at normal temperature and can be molded at high temperature by use of existing molding equipment (because of the hard segment of the molecules) in the same manner as common thermoplastic resins.

For the elastomer (B) of the invention which has water vapor permeability, various elastomers having water vapor permeability my be used.

An elastomer can be determined to have water vapor permeability if it is acknowledged to have water vapor permeability when tested by a testing method based on JIS Z0208, for example. Those elastomers whose sample in the form of film 30 μm in thickness shows water vapor permeability of not less than 2,000 $g/m^2$.d as 30 μm thickness film, preferably not less than 3,000 $g/m^2$.d as 30 μm thickness film, are used preferably in the invention.

Preferable examples of the thermoplastic elastomer having water vapor permeability include urethane-based elastomer, polyester-based elastomer and polyamide-based elastomer.

The elastomer having water vapor permeability may be selected from among those elastomers shown specifically which conform to the requirements of water vapor permeability as described above. Consequently, the elastomer having water vapor permeability can be selected and adopted easily.

As urethane-based elastomer, thermoplastic elastomer is preferable. An example of thermoplastic elastomer is a block copolymer of the polyurethane, as the hard segment, obtained through reaction between short-chain polyol (molecular weight: 60 to 600) and diisocyanate and the polyurethane, as the soft segment, obtained through reaction between long-chain polyol (molecular weight: 600 to 4,000) and diisocyanate. Examples of diisocyanate include toluene diisocyanate and diphenyl methane diisocyante, and examples of short-chain polyol include ethylene glycol, 1,3-propylene glycol and bisphenol A.

Examples of urethane-based thermoplastic elastomer include a product (polyether polyurethane) obtained by addition-polymerizing diisocyanate to polylactone ester polyol, such as polycaprolactone glycol, in the presence of short-chain polyol; a product (polyester polyurethane) obtained by addition-polymerizing diisocyanate to adipate polyol, such as poly(ethylene-1,4-adipate)glycol and poly(butylene-1,4-adipate), in the presence of short-chain polyol; and a product obtained by addition-polymerizing diisocyanate to polytetramethylene glycol formed by the ring opening of tetrahydroflan in the presence of short-chain polyol.

Examples of commercially available urethane-based thermoplastic elastomers include Vulcoran (available from Bayer), Chemgum SL (available from Goodyear), Adiprene (available from DuPont) and Valcaprene (available from ICI). (All of these are tradenames.)

An example of polyester-based elastomer is a block copolymer comprising the structural unit represented by the following formula (I) which is derived from aromatic polyester as the hard segment and the structural unit represented by the following formula (II) which is derived from aliphatic polyether as the soft segment, with these structural units being block-copolymerized.

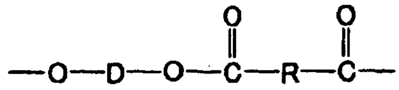

(I)

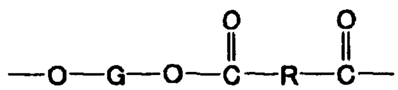

(II)

In the above formulas, D represents a bivalent residue of diol having molecular weight of approximately not more than 250 from which two hydroxyl groups have been removed; R represents a bivalent residue of dicarboxylic acid having molecular weight of approximately not more than 300 from which two carboxyl groups have been removed; and G represents a bivalent residue of poly(alkylene oxide)glycol having average molecular weight of approximately 400 to 3500 from which the hydroxyl groups at both ends have been removed. Here, the amount of the ethylene oxide that is inserted into the structural unit represented by the formula (II), namely copolyether ester of poly(alkylene oxide) glycol, is approximately 25 to 68 wt% to the total weight of copolyether ester. In the invention, especially the aforesaid aromatic polyether is preferably tetramethylene terephthalate, and the aforesaid aliphatic polyether is preferably alkylene ether terephthalated. A specific example is polybutylene terephthalate/polytetramethylene ether glycol block copolymer.

An example of commercially available polyester-based elastomer is Hytorel (tradename; available from Du-Pont).

An example of polyamide-based elastomer is multi block copolymer using polyamide as the hard segment and diol of polyester or polyol low glass transition temperature as the soft segment. Here examples of the polyamide component include nylon 6, nylon 66, nylon 610, nylon 11 and nylon 12. Out of these, nylon 6 and nylon 12 are preferable. poly(oxytetramethylene)glycol and poly(oxypropylene)glycol. Examples of polyester diol include poly(ethylene-1,4-adipate)glycol , poly(butylene-1,4-adipate)glycol and polytetramethylene glycol. A specific example is nylon 12/polytetramethylene glycol block copolymer.

There are such commercially available polyamide-based elastomers as Diamid (Daicel-Hüls) and PEBAX (Atochem). (All of these are tradenames.)

Out of these, urethane-based elastomer and polyester-based elastomer are preferable in that they have excellent water vapor permeability. Urethane-based elastomer having stretchability as well as water vapor permeability is especially preferable.

Further, in the invention, coloring agents, heat stabilizers, lubricants, nucleating agents, other polymers, etc. may be compounded to the aforesaid elastomer materials as required to such extent that the purpose of the invention is not frustrated.

Ethylene polymer (C) containing a polar group

In the invention, the resin of the resin layer formed between the layer of the elastomer (B) having water vapor permeability and the nonwoven fabric (A) is a layer containing the ethylene polymer (C) containing a polar group. The layer of the ethylene polymer (C) containing a polar group functions not only as the adhesive resin layer between the elastomer (B) layer and the nonwoven fabric (A) but also the layer which follows the expansion and contraction of the elastomer (B) layer and the nonwoven fabric (A), imparting stretchability to the nonwoven fabric laminate.

The ethylene polymer (C) containing a polar group which is used in the invention may be either a copolymer of ethylene and a monomer containing a polar group or a polymer obtaining by modifying ethylene polymer with an unsaturated compound containing a polar group.

The copolymer of ethylene and a monomer containing a polar group may be obtained, for example, by subjecting ethylene and a monomer containing a polar group to radical polymerization reaction at high temperature and under high pressure in the presence of an organic peroxide or oxygen.

In the invention, examples of the monomer containing a polar group include ethyl acrylate, methacylic acid, methyl methacrylate, vinyl acetate and vinyl chloride. Out of these, one type selected from the group of ethyl acrylate, methacrylic acid, methyl methacrylate and vinyl acetate is preferable, and especially ethyl acrylate is preferable in that it shows excellent heat resistance and makes high-temperature processing possible.

In the invention, an ethylene/ethyl acrylate copolymer (hereinafter sometimes referred to as "EEA") may be used as the ethylene polymer (C) containing a polar group. The ethyl acrylate content of such copolymer is normally 10 to 40 wt%, preferably 15 to 30 wt%, from a viewpoint of adhesion, cost and water vapor permeability. Moreover, the melt flow rate (at 190°C ) is normally 2 to 300 g/10 min; preferably 50 to 300 g/10 min; more preferably 100 to 300 g/10 min in the case using the meltblown process; preferably 2 to 30 g/10 min; more preferably 5 to 15 g/10 min in the case making the porous film; preferably 5 to 50 g/10 min; more preferably 10 to 30 g/10 min in the case using the co-extrusion process. If the ethyl acrylate content and the melt flow rate are in these ranges, a copolymer showing excellent moldability and interlaminar strength can be obtained.

Examples of commercially available products of such copolymer include Evaflex ™ EEA (Grade A-707, for example) available from DuPont-Mitsui Polychemicals). In the invention, an ethylene/vinyl acetate copolymer (hereinafter referred sometimes to as "EVA") may be used for the ethylene polymer (C) containing a polar group. The vinyl acetate content of the aforesaid copolymer is normally 10 to 40 wt %, preferably 3 to 10 wt %, by reason of adhesion, cost and water vapour permeability. Further, the melt flow rate (at 190°C ) is normally 2 to 300 g/10 min; preferably 50 to 300 g/10 min; more preferably 100 to 300 g/10 min in the case using the meltblown process; preferably 2 to 30 g/10 min; more preferably 5 to 15 g/10 min in the case making the porous film; preferably 5 to 50 g/10 min; more preferably 10 to 30 g/10 min in the case using the co-extrusion process. If the vinyl acetate content and the melt flow rate are in these ranges, a copolymer showing excellent moldability and interlaminar strength can be obtained.

An example of commercially available ethylene/vinyl acetate copolymer is Evaflex ™ (grade P-2807, for example) available from Du-Pont-Mitsui Polychemicals Co.

In the invention, as the ethylene polymer (C) containing a polar group, an ethylene/methacrylic acid copolymer may be used. The methacrylic acid content of the aforesaid copolymer is normally 10 to 40 wt %, preferably 3 to 10 wt %, by reason of adhesion, cost and water vapour permeability. Further, the melt flow rate (at 190°C ) is normally 2 to 300 g/10 min; preferably 50 to 300 g/10 min; more preferably 100 to 300 g/10 min in the case using the meltblown process; preferably 2 to 30 g/10 min; more preferably 5 to 15 g/10 min in the case making the porous film; preferably 5 to 50 g/10 min; more preferably 10 to 30 g/10 min in the case using the co-extrusion process. If the methacrylic acid content and the melt flow rate are in these ranges, a copolymer showing excellent moldability and interlaminar strength can be obtained.

An example of commercially available ethylene/methacrylic acid copolymer is Nucrel™ available from DuPont-Mitsui Polychemicals Co.

In the invention, as the ethylene polymer (C) containing a polar group, ethylene-based ionomer resin may be used. As used herein, an ionomer resin means a thermoplastic resin whose high-molecular-weight main chain contains a small amount of an ion group in its side chain, terminal chain or itself. The ethylene-based ionomer resin of the invention is a metal salt of a copolymer of ethylene and unsaturated carboxylic acid.

As an example of the commercially available material, Himilan™ (available from DuPont-Mitsui Polychemicals) using methacrylic acid as acid monomer may be cited.

In the invention, as the ethylene polymer (C) containing the polar group, polymers modified with an unsaturated compound containing the polar group may be used. Similar examples to the ones mentioned above may be cited as examples of such ethylene polymer, and a copolymer of ethylene and α-olefin which contains a relatively large amount of α-olefin may be cited as an example. These ethylene polymers may be of high-crystallinity or low-crystallinity. The aforesaid unsaturated compound containing the polar group that may be used in modifying ethylene polymer may be a monomer containing the polar group that is to be copolymerized with ethylene, or any other unsaturated compounds containing the polar group. Preferable examples of such unsaturated compound are unsaturated carboxylic acid and its anhydride.

Examples of such unsaturated carboxylic acid include unsaturated carboxylic acid such as maleic acid, fumaric acid, tetrahydrophthalic acid, itaconic acid, citraconic acid, crotonic acid, isocrotonic acid, Nadic Acid (a tradename of endo-cis-bicyclo[2,2,1]hepto-5-en-2,3-dicarboxylic acid), acrylic acid and methacrylic acid; or their derivatives, including anhydride, imide, amide and ester of the aforesaid unsaturated carboxylic acid, specifically maleimide, maleic anhydride, citraconic anhydride, maleic monomethyl and glycidyl maleate, for example. Out of these, unsaturated carboxylic acid or its acid anhydride are preferable. Especially maleic acid, Nadic Acid and their acid anhydrides are preferable.

In the invention, a copolymer of ethylene and a monomer containing the polar group which has been modified with any of the aforesaid unsaturated compounds containing the polar group may be used as the ethylene polymer (C) containing the polar group. For example, the aforesaid copolymer of ethyl acrylate may be modified with unsaturated carboxylic acid. For example, a modified resin obtained by modifying the aforesaid copolymer of ethyl acrylate with unsaturated carboxylic acid may be used. Use of such modified resin is preferable because it enables the interlaminar strength between water-vapour-permeable resin and non-woven fabric to be improved, compared with the case of using unmodified resin. The graft monomer content, showing the modification rate, of the modified resin used in the invention is normally 0.01 to 10 wt %, preferably 0.1 to 3 wt%.

Resins obtained by modifying EEA with maleic anhydride are available commercially.
In the invention, the ethylene polymer (C) having the polar group may also be used with the aforesaid elastomer (B) having water vapour permeability, a tacky resin, an inorganic filler or a foaming agent added to it as required.
Examples of such tacky resin include alicyclic saturated hydrocarbon resin and terpene resin. Moreover, examples of such inorganic filler include calcium carbonate, talc, clay and barium sulfate.
The additions amounts of these additives based on 100 parts by weight of the resin (C) are normally 5 to 50 parts by weight, preferably 5 to 20 parts by weight, for the elastomer (B) having water vapor permeability, normally 0.5 to 20 parts by weight, preferably 0.5 to 10 parts by weight, for the tacky resin, and normally 10 to 60 parts by weight, preferably 20 to 40 parts by weight, for the inorganic filler. In addition to the aforesaid additives, other additives such as coloring agents, heat stabilizers, lubricants, nucleating agents and other resins may be added.

Non woven fabric laminates

The nonwoven fabric laminate may be obtained by laminating the aforesaid nonwoven fabric (A) on one or both sides of a film of the aforesaid elastomer (B) having water vapor permeability through a layer of the aforesaid ethylene polymer (C) containing the polar group.
The layer of the aforesaid ethylene polymer (C) containing the polar group may be formed in such manner that it has pores partially but preferably from the viewpoint of adhesion and processability formed in the form of film. It is recommended that the layer of the ethylene polymer (C) containing the polar group be formed to a basis weight of not more than 10 g/m$^2$ as a method for preventing the water vapor permeability of the nonwoven fabric laminate from declining due to the provision of the layer of the ethylene polymer (C) containing the polar group. The layer of the ethylene polymer (C) containing the polar group is formed to a basis weight of preferably not more than 5 g/m$^2$, more preferably not more than 3 g/m$^2$, much more preferably 1 to 3 g/m$^2$. This enables adequate water vapor permeability and adhesion to be obtained.
In the invention, the layer of the ethylene polymer (C) containing the polar group may be made a porous layer. Making the layer of the ethylene polymer (C) containing the polar group a porous layer produces the desirable effects of not only functioning as an adhesive resin layer between the nonwoven fabric (A) and the elastomer (B) having water vapor permeability but also maintaining the water vapor permeability of the nonwoven fabric laminate and exhibiting stretchability that allows the layer of the ethylene polymer (C) containing the polar group to follow the expansion and contraction of the nonwoven fabric (A) and the elastomer (B) having water vapor permeability. Examples of such porous layer include nonwoven fabric and porous film.
An example of such porous film is a film so formed that it has pores partially. The size of such pores is normally 0.5 to 20 μm, preferably 1 to 5 μm. Further, the void rate as defined below is normally 10 to 50%. The void rate as used herein is defined by the following formula:

Void rate (%)

= [(Density of raw material resin - apparent density of film)/

(density of raw material resin)] x 100

As the method for laminating the layers of the nonwoven fabric (A) and the ethylene polymer (C), the method in which the nonwoven fabric (A), a film of the elastomer (B) and a film of the ethylene polymer (C) containing the polar group are formed in advance and the nonwoven fabric (A), a film of the ethylene polymer (C) containing the polar group and a film of the elastomer (B) are laid on top of another in this order and bonded under pressure by means of embossing rolls, for example, and the method in which a layer of the ethylene polymer (C) containing the polar group is inserted and bonded between the films of nonwoven fabric (A) and the elastomer (B) formed in advance by means of coating or extrusion may be adopted, but these methods present problem in processability due to the thinness of the layer of the ethylene polymer (C).
In the invention, a preferable method for laminating the layers of the nonwoven fabric(A) and the ethylene polymer (C) containing the polar group is the method in which a film of the ethylene polymer (C) containing the polar group and a film of the elastomer (B) are coextruded and bonded by lamination to the nonwoven fabric (A) formed in advance. Use of such extrusion lamination method eliminates the step of lamination by use of a hot melt adhesive which has been involved in the conventional process and enables the formation of films and their bonding with the nonwoven fabric to be carried at the same time, simplifying the fabrication process and consequently achieving a cost reduction.
Given below is an explanation of the method using extrusion lamination in accordance with Fig. 1. The elastomer material is fed to extruder 1, and the ethylene/polar-group-containing monomer copolymer is fed to extruder 2. The melted resins melted in the two extruders are sent to multi-layer film die 3 so that a laminated film is formed. On the other hand, a nonwoven fabric produced in advance by any method, for example, by the spunbonding method and stretched when required is sent to nip rolls 6 from feed roll 4. In the meantime, the surface of the nonwoven fabric may be modified by subjecting the nonwoven fabric to corona discharge treatment 5. The nip rolls may be flat rolls, but they can preferably be embossing rolls having embossing protrusions because of the use of embossing rolls improves the softness of the nonwoven fabric. In this case, the embossing area of the embossing rolls is normally 5% to 30%, though it is determined depending on the required interlaminar strength.

The laminated film extruded from Multi-layer Film Die 3 which comprises the elastomer (B) having water vapor permeability and the ethylene polymer containing the polar group is sent between Nip Roll 6 and Chill Roll 7 before it is cooled and solidified and passed between the two rolls in such manner that the adhesive resin layer comes in contact with the nonwoven fabric, so that they will be bonded under pressure. At the same time, the laminated film is cooled and solidified by means of the chill roll, and the bonded nonwoven fabric laminate is taken up onto take-up roll 8.

Fig. 2 shows the nonwoven fabric laminate thus obtained. In Fig. 2, "a" represents the thermoplastic elastomer (B) having water vapor permeability, "b" represents the nonwoven fabric (A), and the layers of (A) and (B) are laminated through the adhesive resin (C), represented by "c", which comprises the ethylene polymer containing the polar group.

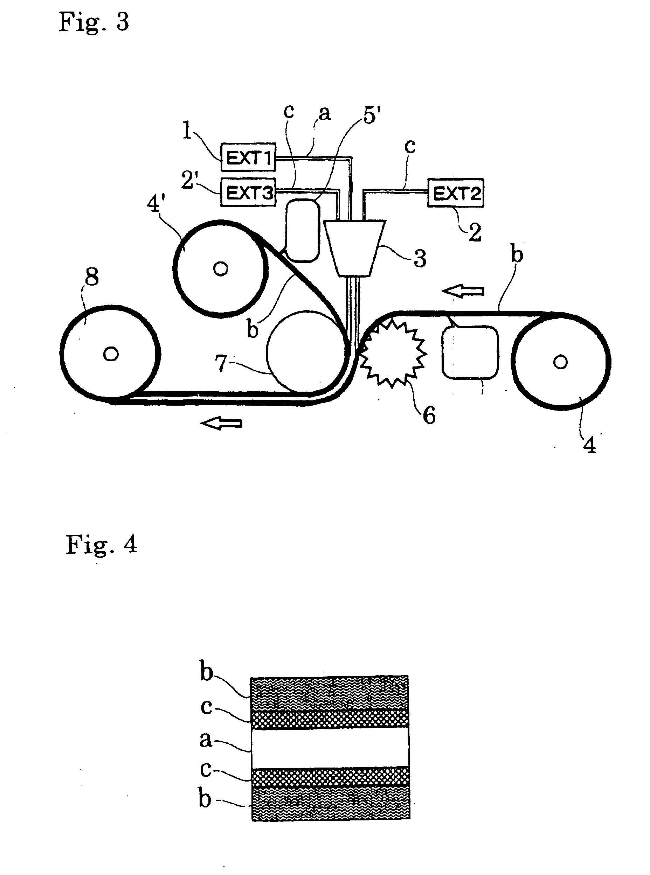

Fig. 3 illustrates an example of extrusion lamination in which the nonwoven fabric (A) is bonded to both sides of a film of the elastomer (B) having water vapor permeability. In addition to Fig. 1, the ethylene polymer (C) containing the polar group is fed to Extruder 2'. Furthermore, another nonwoven fabric is fed from Feed Roll 4'. The nonwoven fabric from Feed Roll 4' is subjected to corona discharge treatment by means of 5'. Fig. 4 shows a nonwoven fabric laminate obtained by the method as shown in Fig. 3.

Lamination may be carried out similarly in laminating a stretchable nonwoven fabric (A) to one side of a film of the elastomer (B) and another film to the other side of the film of the elastomer (B).

If the elastomer (B) is sticky, blocking may take place on the cooling chill roll in the process of producing the aforesaid nonwoven fabric laminate. It is proposed as a method for preventing blocking on the chill roll that a separate peeling film be provided (in Japanese Patent Publication SHO 58-69049, for example). However, this method poses problems of a rise in the cost and the discharge of unnecessary waste materials.

The inventors recommend the method for preventing blocking by coating a non-sticky material onto the chill roll.

Specific example of the method for coating a non-sticky material onto the chill roll include the method of coating synthetic resin including fluorine resin such as polytetrafluoroethylene (PTFE) and others, and the method of spray-coating ceramic.

Polytetrafluoroethylene (PTFE) is preferable as such synthetic resin used for the coating. Such synthetic resin may be mixed with a filler such as ceramic at the time of the coating. The mixing of ceramic improves the wear resistance of the coating. The thickness of the coating is preferably 30 to 50 µm.

In the method of spray-coating ceramic, an appropriate ceramic may be selected for the ceramic, but preferable examples include WC and $Cr_2C_3$. Particularly WC may be used preferably. The thickness of the spray-coating is preferably 100 to 200 µm, and its surface roughness (Ra) is preferably 1 to 10.

An embodiment in which such resin or a silicone is coated additionally on the chill roll spray-coated with ceramic provides a coating with improved wear resistance and therefore is a preferable embodiment of such non-sticky coating. Polytetrafluoroethylene (PTFE) may be cited as a preferable synthetic resin used in this embodiment, and silicone rubber may be cited as a preferable silicone used in this embodiment.

Given below is an explanation of an embodiment of the invention in which a porous layer is used as the layer of the ethylene polymer (C) containing the polar group.

A method in which a layer of the porous material comprising the ethylene polymer (C) containing the polar group and a film of the elastomer (B) having water vapor permeability are formed in advance, the layer of porous material is laid on and bonded under pressure by means of embossing rolls, for example, with the nonwoven fabric (A), and then the film of the elastomer (B) is pressed onto the porous material side, heated to a temperature level required for their bonding and at the same time bonded under pressure; and a method in which a film of the thermoplastic elastomer, a porous material and the nonwoven fabric (A) are laid on top of the other in this order and then bonded in the same manner may be adopted. In the invention, if the porous material is a nonwoven fabric, a preferable method is a one in which a meltblown nonwoven fabric layer comprising the ethylene polymer (C) containing the polar group is formed on the nonwoven fabric (A) and then a film of the elastomer (B) is extruded and bonded onto it by extrusion lamination. Use of the extrusion lamination method eliminates the step of lamination by use of a hot melt adhesive which has been involved in the conventional process and enables the formation of films and their bonding with the nonwoven fabric to be carried at the same time, simplifying the fabrication process and consequently achieving a cost reduction.

Specifically, the nonwoven fabric (A) is sent onto the cylindrical drum having apertures whose inside is in a

state of reduced pressure, and the ethylene polymer (C) containing polar group in a melted state is sprayed from the meltblown spinning nozzle installed over the cylindrical drum, so that a meltblown nonwoven fabric layer is formed on the nonwoven fabric (A). At this time, as the meltblown nonwoven fabric fiber reaches the nonwoven fabric (A) before the resin solidifies completely, it is fused with the fiber of the nonwoven fabric (A). This makes it unnecessary to bond the nonwoven fabric (A) and the meltblown nonwoven fabric layer again. The diameter of the fiber of the meltblown nonwoven fabric layer is preferably 0.1 to 20 µm, more preferably 5 to 15 µm, and its basis weight is preferably 2 to 20 g/$m^2$, more preferably 3 to 10 g/$m^2$. If the basis weight is in this range, the laminate shows interlaminar strength of not less than 20 g/inch, while retaining satisfactory stretchability.

Next, a film of the elastomer (B) is extruded and bonded onto the meltblown nonwoven fabric layer side of the nonwoven fabric sheet by the extrusion lamination method.

In the case of another extrusion lamination example, the aforesaid nonwoven fabric sheet may be bonded with both sides of a film of the elastomer (B) in the same manner. It is also possible to laminate the nonwoven fabric sheet onto the one side of the elastomer (B) film and another film onto the other side.

In the invention, the basis weight of the film of the elastomer (B) is selected depending on the strength required for individual applications. For example, for back sheets for use for disposable diapers, the basis weight is preferably 5 to 30 g/$m^2$, more preferably 8 to 15 g/$m^2$. As the basis weight of the elastomer (B) film becomes too high, the water vapor permeability becomes inadequate; while as the basis weight of the elastomer (B) film becomes too low, the strength becomes low. This may present a problem in use.

Further, the basis weight of the layer of the polar-group-containing ethylene polymer (C) is preferably not more than 10 g/$m^2$, more preferably not more than 5 g/$m^2$, further preferably not more than 3, especially preferably 1 to 3 g/$m^2$. If the basis weight is in this range, adequate water vapor permeability is obtained, and the interlaminar strength is not less than 20 g/2.54 cm (g/inch).

The water-vapor-permeable nonwoven fabric laminate of the invention is preferably not less than 1,000 g/$m^2$. day in water vapor permeability, and not less than 1,000 mm Aq, more preferably not less than 1,500 mm Aq, in waterproofness. This nonwoven fabric laminate shows excellent water vapor permeability and waterproofness.

Furthermore, the interlaminar strength between the nonwoven fabric (A) and the elastomer (B) having water vapor permeability of the water-vapor-permeable nonwoven fabric laminate of the invention is preferably not less than 20 g/2.54 cm (g/inch), more preferably not less than 40 g/inch. If the interlaminar strength is not less than 20 g/2.54 cm (g/inch), the water-vapor-permeable nonwoven fabric laminate of the invention shows excellent water vapor permeability and waterproofness and high interlaminar strength. Because of this, the water-vapor-permeable nonwoven fabric laminate is suitable for use for such applications as medical materials, sanitary materials and industrial materials. Specific examples of applications for the water-vapor-permeable nonwoven fabric laminate of the invention include protective covers, packaging materials, jackets, underwear, sanitary goods, disposable diapers, roofing materials and wallpaper.

## EXAMPLES

In the following examples are described several preferred embodiments to illustrate the invention. However, it is to be understood that the invention is not intended to be limited to the specific embodiments.

The methods used to determine the tensile properties, residual stress after elongation and interlaminar strength of the nonwoven fabrics obtained in these examples and comparative examples are as follows:

(1) Interlaminar strength:

Each layer at one end of a dumbbell-shaped specimen 25 mm in width of the nonwoven fabric laminate was partially peeled in the longitudinal direction in advance. Both ends of each peeled end were gripped by the chucks of the tester (Model 2005 available from Isotesco) in the T-shaped form (180° peeling) so that the chuck distance was 50 mm, and the adhesion between the layers was determined by peeling the layers at a peeling rate of 100 mm/minute.

(2) Water vapor permeability:

The water vapor permeability was determined in accordance with the cup method of JIS Z 0208. The mass of the water vapor that permeated through the sample having a water vapor permeable area of not less than 25 $cm^2$ for 30 minutes in an atmosphere at a temperature of 40°C and a relative humidity of 90% was measured. The measured value was converted to a value for 1 $m^2$ of the sample as measured for 24 hours. Further, the atmosphere on the side of excess water vapor was made dry by use of a dehumidifying agent.

(3) Waterproofness:

The waterproofness was determined in accordance with JIS L 1072A (low water pressure method). Four each of test specimens approx. 15 x 15 cm in size were taken and mounted on the waterproofness testing equipment (available Tester Industry) in such manner that the surfaces of the specimen would come in contact with water. Water pressure was applied to the test specimen by raising the spirit level containing water at normal temperature at a rate of 60 ± 3 cm/min. or 10 ± 0.5 cm/min. At this time, the water level at which the water leaked at three spots on the other side of the test specimen was measured, the waterproofness was determined from the pressure at that time.
MFR of polymer was measured at the temperature indicated in each case with the load of 2.16 Kg in accordance with ASTM D1238.

Production Example 1

A spunbonded nonwoven fabric (1) having the basis weight of 18 g/$m^2$ (denier of the component fiber: 2.7 d; tensile strength (MD): 1,500 g/25 mm; tensile strength (CD): 500 g/25 mm) was produced by melt-spinning a conjugate fiber of the concentric core-sheath type whose core comprises the propylene/ethylene random copolymer and whose sheath comprises the ethylene/1-butene random copolymer (weight ratio between core:sheath = 1:4) and then causing the conjugate fiber to stack on the collecting surface. Said propylene/ethylene random copolymer has the density of 0.90 g/$cm^3$, the MFR of 50 g/10 minutes (at 230°C) and the ethylene content of 4.7 mol%. And said ethylene/1-butene random copolymer has the density of 0.948 g/$cm^3$, the MFR of 30 g/10 minutes (at 190°) and the 1-butene content of 4.0 mol%.

Production Example 2

A spunbonded nonwoven fabric (2) having the basis weight of 20 g/$m^2$ (denier of the component fiber: 2.4 d; tensile strength (MD): 1,900 g/25 mm; tensile strength (CD): 800 g/25 mm) was produced by melt-spinning a fiber from a propylene/ethylene random copolymer having the density of 0.90 g/$cm^3$, the MFR of 50 g/10 minutes (at 230°) and the ethylene content of 4.7 mol%, and then causing the conjugate fiber to stack on the collecting surface.

Example 1:

A laminated film of a polyester-based thermoplastic elastomer and an ethylene/ethyl acrylate copolymer (basis weight of elastomer film and ethylene/ethyl acrylate copolymer: 12 g/$m^2$ and 3 g/$m^2$, respectively) was produced by use of a multi-film die (temperature: 220°C). The polyester-based thermoplastic elastomer (polyester-based TPE (1); Hytrel™ 3548 available from DuPont; water vapor permeability of the resin: 3,620 g/$m^2$.d as 30 μm thickness film) was extruded from the first extruder (molding temperature pattern: 200°C/240°C/230°C). And the ethylene/ethyl acrylate copolymer (EEA (1); Evaflex™ EEA A-709 available from DuPont-Mitsui Polychemicals (MFR: 25 g/10 min (at 190°C)) extruded from the second extruder (molding temperature pattern: 180°C/210°/200°C). Output ratio of the first extruder/ the second extruder was 12/3).
On the other hand, the nonwoven fabric (1) produced as described above was fed at 30 m/min., subjected to corona discharge treatment at 30 W/$m^2$, and sent between the nip and chill rolls, so that the nonwoven fabric (1) was laminated to the aforesaid laminated film and consequently a nonwoven fabric laminate was produced. The roll conditions were such that the temperature was 40°C and the speed was 30 m/min. Results of the laminate are shown in Table 1.
In Example 1 and subsequent Examples, a roll to which polytetrafluoroethylene has been coated by baking to a thickness of 40 μm was used.

Example 2:

A nonwoven fabric laminate was produced by the same method as described in Example 1 except that the basis weight of the polyester-based thermoplastic elastomer (polyester-based TPE (1)) was 15 g/$m^2$. Results of the laminate are shown in Table 1.

Example 3:

A nonwoven fabric laminate was produced by the same method as described in Example 1 except that a polyester-based thermoplastic elastomer (polyester-based TPE (2); Hytrel™ 8171 available from DuPont; water vapor permeability of the resin: 5300 g/$m^2$d as 30μm thickness film) was used in place of the polyester-based TPE (1). Results

of the laminate are shown in Table 1.

Example 4:

A laminated film of a polyester-based thermoplastic elastomer and an ethylene/ethyl acrylate copolymer(basis weight of the elastomer film and the ethylene/ethyl acrylate copolymer: 8 g/m$^2$; and 2 g/m$^2$,respectively) was produced by use of a multi-film die (temperature: 220°C). The polyester-based thermoplastic elastomer (polyester-based TPE (2); Hytrel™ 8171 available from DuPont; water vapor permeability of the resin: 5,300 g/m$^2$d as 30 µm thickness film) was extruded from extruder 1(molding temperature pattern: 200°C/240°C/230°C) and the ethylene/ethyl acrylate copolymer (EEA (1); Evaflex™ EEA A-709 available from DuPont-Mitsui Polychemicals (MFR: 25 g/10 min. (at 190°C)) was extruded from extruder 2(molding temperature pattern: 180°C/210°/200°C). Output ratio of extruder 1/extruder 2 was 8/2.

On the other hand, the nonwoven fabric (2) produced as described above was fed at 30 m/min., subjected to corona discharge treatment at 30 W/m$^2$, and sent between the nip and chill rolls, so that the nonwoven fabric (2) was laminated to the aforesaid laminated film and consequently a nonwoven fabric laminate was produced. The roll conditions were such that the temperature was 40°C and the speed was 30 m/min. Results of the laminate are shown in Table 1.

Table 1

| | | Example 1 | Example 2 | Example 3 | Example 4 |
|---|---|---|---|---|---|
| Elastomer(B) | Material | Polyester-based TPE(1) | Same as left | Polyester-based TPE(2) | Same as left |
| | Basis weight (g/m$^2$) | 12 | 15 | 12 | 8 |
| Ethylene polymer (C) containing polar group | Material | EEA(1) | Same as left | Same as left | Same as left |
| | Basis weight (g/m$^2$) | 3 | 3 | 3 | 2 |
| Nonwoven fabric (A) | Basis weight (g/m$^2$) | 23 | 23 | 23 | 20 |
| Interlaminar strength (g/in.) | | 80 | 120 | 80 | 80 |
| Water vapor permeability (g/m$^2$·day | | 3800 | 3000 | 4200 | 6000 |
| Waterproofness (mm Aq) | | >2000 | >2000 | >2000 | >2000 |

Comparative Example 1:

An elastomer film having the basis weight of 30 g/m$^2$ was produced by use of a film die (temperature: 220°C) by extruding the aforesaid polyester-based TPE (1) by means of an extruder (molding temperature pattern: 200°C/240°C/230°C). On the other hand, the nonwoven fabric (1) produced as described above was fed at 30 m/min., subjected to corona discharge treatment at 30 W/m$^2$, and sent between the nip and chill rolls, so that the nonwoven fabric (1) was laminated to the aforesaid elastomer film and consequently a nonwoven fabric laminate was produced. The roll conditions were such that the temperature was 40°C and the speed was 30 m/min. Results of the laminate are shown in Table 2.

Comparative Example 2:

A nonwoven fabric laminate was produced by the same method as described in Comparative Example 1 except that the basis weight of the polyester-based TPE (1) was 15 g/m$^2$. Results of the laminate are shown in Table 2.

Comparative Example 3:

A film having the basis weight of 8 g/m$^2$ was produced by use of a film die (temperature: 220°C) by blending the aforesaid polyester-based TPE (1) and the aforesaid EEA (1) and extruding the blend by means of an extruder (molding temperature pattern: 200°C/230°C/220°C).

On the other hand, the nonwoven fabric (1) produced as described above was fed at 30 m/min., subjected to

corona discharge treatment at 30 W/$m^2$, and sent between the nip and chill rolls, so that the nonwoven fabric (1) was laminated to the aforesaid extruded film and consequently a nonwoven fabric laminate was produced. The roll conditions were such that the temperature was 40°C and the speed was 30 m/min. Results of the laminate are shown in Table 2.

Comparative Example 4:

An attempt was made to produce a nonwoven fabric laminate by the same method as described in Comparative Example 3 except that the aforesaid polyester-based TPE (1) and the aforesaid EEA (1) were blended at a weight ratio of 5/5. However, no normal film was formed due to poor compatibility.

Comparative Example 5:

A film having the basis weight of 12 g/$m^2$ was produced by use of a film die (temperature: 220°C) by extruding the aforesaid EEA (1) by means of an extruder (molding temperature pattern: 180°C/210°C/200°C). On the other hand, the nonwoven fabric (1) produced as described above was fed at 30 m/min., subjected to corona discharge treatment at 30 W/$m^2$, and sent between the nip and chill rolls, so that the nonwoven fabric (1) was laminated to the aforesaid extruded film and consequently a nonwoven fabric laminate was produced. The roll conditions were such that the temperature was 40°C and the speed was 30 m/min. Results of the laminate are shown in Table 2.

Table 2

| | | Comparative Example 1 | Comparative Example 2 | Comparative Example 3 | Comparative Example 5 |
|---|---|---|---|---|---|
| Elastomer(B) | Material | Polyester-based TPE(1) | Same as left | Polyester-based TPE(1)/EEA (1) | EEA (1) |
| | Basis weight (g/$m^2$) | 30 | 15 | 8 | 12 |
| Nonwoven fabric (A) | Basis weight (g/$m^2$) | 23 | 23 | 23 | 20 |
| Interlaminar strength (g/in.) | | 2 | 1 | 5 | 150 |
| Water vapor permeability (g/$m^2$·day) | | 3300 | 5000 | 6000 | 1200 |
| Waterproofness (mm Aq) | | >2000 | >2000 | >2000 | >2000 |

Example 5:

A laminated film of the aforesaid polyester-based TPE (1) and an ethylene/ethyl acrylate copolymer (EEA (1)) (basis weight of the polyester-based TPE (1) film and the EEA (1): 8 g/$m^2$ and 3 g/$m^2$,) was produced by use of a multi-film die (temperature: 250°C). The aforesaid polyester-based TPE (1) was extruded from extruder 1(molding temperature pattern: 200°C/240°C/230°C) and the ethylene/ethyl acrylate copolymer (EEA (1)) or a mixture thereof with any of the additives shown in Table 3 below as the ethylene polymer (C) containing the polar group was extruded from extruder 2(molding temperature pattern: 180°C/210°C/200°C). Output ratio of extruder 1/extruder 2 was 8/3).

On the other hand, the nonwoven fabric (2) produced as described above was fed at 30 m/min., subjected to corona discharge treatment at 30 W/$m^2$, and sent between the nip and chill rolls, so that the nonwoven fabric (2) was laminated to the aforesaid laminated film and consequently a nonwoven fabric laminate was produced. The roll conditions were such that the temperature was 40°C and the speed was 30 m/min. Evaluation results of the laminates are shown in 5-1 to 5-4 of Table 3.

Table 3

| | Ethylene polymer (C) containing polar group | | Interlaminar strength (g/in.) | Water vapor permeability (g/ $m^2$·day) | Waterproofness (mm Aq) |
|---|---|---|---|---|---|
| | Resin (wt%) | Additive (wt%) | | | |
| 5-1 | EEA(1) (100) | None | 80 | 3000 | >2000 |
| 5-2 | EEA(1) (80) | Polyester-based TPE(2) (20) | 100 | 4000 | >2000 |
| 5-3 | EEA(1) (90) | Tackifier Alcon P125 (10) | 100 | 2800 | >2000 |
| 5-4 | EEA(1) (60) | Calcium Carbonate (40) | 50 | 4000 | >2000 |

Example 6:

A nonwoven fabric laminate was produced by the same method as described in Example 5 except that an ethylene/vinyl acetate copolymer (EVA (1); EVA 150 available from DuPont-Mitsui Polychemicals) and mixtures thereof with the additives shown in Table 4 below were used for the ethylene polymer (C) containing the polar group. Measurement results of the laminates are shown in 6-1 to 6-4 of Table 4.

Table 4

| | Ethylene polymer (C) containing polar group | | Interlaminar strength (g/in.) | Water vapor permeability (g/ $m^2$·day) | Waterproofness (mm Aq) |
|---|---|---|---|---|---|
| | Resin (wt%) | Additive (wt%) | | | |
| 6-1 | EVA(1) (100) | None | 80 | 3000 | >2000 |
| 6-2 | EVA(1) (80) | Polyester-based TPE(2) (20) | 90 | 4000 | >2000 |
| 6-3 | EVA(1) (90) | Tackifier Alcon P125 (10) | 100 | 2800 | >2000 |
| 6-4 | EVA(1) (60) | Calcium Carbonate (40) | 50 | 4000 | >2000 |

Example 7:

A laminated film of the aforesaid polyester-based TPE (1) and an ethylene/methacrylic acid copolymer (basis weight of the polyester-based TPE (1) film and EMA (1): 8 g/$m^2$; and 2 g/$m^2$,respectively) was produced by use of a multi-film die (temperature: 240°C). The aforesaid polyester-based TPE (1) was extruded from extruder 1(molding temperature pattern: 200°C/240°C/230°C) and the ethylene/methacrylic acid copolymer (EMA (1); Nucrel™ AN-4311 available from DuPont-Mitsui Polychemicals) or a mixture thereof with any of the additives shown in Table 5 below as the ethylene polymer (C) containing the polar group was extruded from extruder 2(molding temperature pattern: 180°C/210°C/200°C). Output ratio of extruder 1/ extruder 2 was 8/2).

On the other hand, the nonwoven fabric (2) produced as described above was fed at 30 m/min., subjected to corona discharge treatment at 30 W/$m^2$, and sent between the nip and chill rolls, so that the nonwoven fabric (2) was laminated to the aforesaid laminated film and consequently a nonwoven fabric laminate was produced. The roll conditions were such that the temperature was 40°C and the speed was 30 m/min. Measurement results of the laminates are shown in 7-1 to 7-3 of Table 5.

Table 5

| | Ethylene polymer (C) containing polar group | | Interlaminar strength (g/in.) | Water vapor permeability (g/m$^2$·day) | Waterproofness (mm Aq) |
|---|---|---|---|---|---|
| | Resin (wt%) | Additive (wt%) | | | |
| 7-1 | EMA(1) (100) | None | 90 | 1000 | >2000 |
| 7-2 | EMA(1) (80) | Polyester-based TPE(2) (20) | 100 | 1500 | >2000 |
| 7-3 | EMA(1) (60) | Calcium Carbonate (40) | 60 | 2000 | >2000 |

Example 8:

A nonwoven fabric laminate was produced by the same method as described in Example 7 except that an ethylene/methacrylic acid copolymer metal salt (Ionomer Himilan™ available from DuPont-Mitsui Polychemicals) or a mixture thereof with any of the additives shown in Table 6 below was used as the ethylene polymer (C) containing the polar group and that the rate of feed of the nonwoven fabric and the roll conditions were 50 m/min. Measurement results of the laminates are shown in 8-1 to 8-3 of Table 6.

Table 6

| | Ethylene polymer (C) containing polar group | | Interlaminar strength (g/in.) | Water vapor permeability (g/m$^2$·day) | Waterproofness (mm Aq) |
|---|---|---|---|---|---|
| | Resin (wt %) | Additive (wt %) | | | |
| 8-1 | Ionomer (100) | None | 90 | 1000 | >2000 |
| 8-2 | Ionomer (80) | Polyester-based TPE(2) (20) | 100 | 1500 | >2000 |
| 8-3 | Ionomer (60) | Calcium Carbonate (40) | 60 | 2000 | >2000 |

Example 9:

A nonwoven fabric laminate was produced by the same method as described in Example 5 except that an ethylene/ethyl acrylate copolymer modified with maleic anhydride (modified EEA; HRR-AR201 available from DuPont-Mitsui Polychemicals) and mixtures thereof with the additives shown in Table 7 below were used for the ethylene polymer (C) containing the polar group. Measurement results of the laminates are shown in 9-1 to 9-4 of Table 7.

Table 7

| | Ethylene polymer (C) containing polar group | | Interlaminar strength (g/in.) | Water vapor permeability (g/m$^2$·day) | Waterproofness (mm Aq) |
|---|---|---|---|---|---|
| | Resin (wt%) | Additive (wt%) | | | |
| 9 - 1 | Modified EEA (100) | None | 120 | 3000 | >2000 |
| 9 - 2 | Modified EEA (80) | Polyester-based TPE(2) (20) | 120 | 4000 | >2000 |
| 9 - 3 | Modified EEA (90) | Tackifier Alcon P125 (10) | 130 | 2800 | >2000 |
| 9 - 4 | Modified EEA (60) | Calcium Carbonate (40) | 80 | 4000 | >2000 |

Example 10:

A nonwoven fabric laminate was produced by the same method as described in Example 5 except that polyethylene modified with maleic anhydride (modified PE; ADMER™ AT-951 available from Mitsui Chemicals) and mixtures thereof with the additives shown in Table 8 below were used for the ethylene polymer (C) containing the polar group. Measurement results of the nonwoven fabric are shown in Table 8.

Table 8

| | Ethylene polymer (C) containing polar group | | Interlaminar strength (g/in.) | Water vapor permeability (g/$m^2$·day) | Waterproofness (mm Aq) |
|---|---|---|---|---|---|
| | Resin (wt%) | Additive (wt%) | | | |
| 10 | Modified PE (80) | Polyester-based TPE(2) (20) | 100 | 1200 | >2000 |

Example 11:

A laminated film of a polyurethane-based thermoplastic elastomer and an ethylene/ethyl acrylate copolymer (basis weight of the elastomer film and ethylene/ethyl acrylate copolymer: 12 g/$m^2$ and 3 g/$m^2$ respectively) was produced by use of a multi-film die (temperature: 220°C). The polyurethane-based thermoplastic elastomer (Polyurethane-based TPE (1)); Lezamine™ P210 available from Dainichiseika Color & Chemicals Mfg. Co.; water vapor permeability of the resin: 4,000 g/$m^2$.d as 30 μm thickness film) was extruded from extruder 1 (molding temperature pattern: 200°C/240°C/230°C) and the ethylene/ethyl acrylate copolymer (EEA (1); Evaflex® EEA A-709 (MFR of 25 g/10 minutes (at 190°C)) available from DuPont-Mitsui Polychemicals) as the ethylene polymer (C) containing the polar group was extruded from extruder 2(molding temperature pattern: 180°C/210°C/200°C). Output ratio of extruder 1/extruder 2 was 12/3.

On the other hand, the nonwoven fabric (1) produced as described above was fed at 30 m/min., subjected to corona discharge treatment at 30 W/$m^2$, and sent between the nip and chill rolls, so that the nonwoven fabric (1) was laminated to the aforesaid laminated film and consequently a nonwoven fabric laminate was produced. The roll conditions were such that the temperature was 40°C and the speed was 30 m/min. Evaluation results of the laminates are shown in Table 9.

Example 12:

A nonwoven fabric laminate was produced by the same method as described in Example 11 except that the basis weight of the polyurethane-based thermoplastic elastomer (Polyurethane-based TPE (1)) was 15 g/$m^2$. Evaluation results of the laminate are shown in Table 9.

Table 9

| | | Example 11 | Example 12 |
|---|---|---|---|
| Elastomer (B) | Material | Polyurethane-based TPE(1) | Same as left |
| | Basis weight (g/$m^2$) | 12 | 15 |
| Ethylene polymer (C) containing polar group | Material | EEA(1) | Same as left |
| | Basis weight (g/$m^2$) | 3 | 3 |
| Nonwoven fabric (A) | Basis weight (g/$m^2$) | 23 | 23 |
| Interlaminar strength (g/in.) | | 80 | 90 |
| Water vapor permeability (g/$m^2$·day) | | 3000 | 2800 |
| Waterproofness (mm Aq) | | >2000 | >2000 |

Example 13:

A mixture of 100 parts by weight of a maleic acid-modified blended resin (MFR: 28 g/10 min(at 190°C); acid-modification rate: 0.1 wt%) obtained by blending ethylene- α-olefin copolymer Tafmer™ A (available from Mitsui Chemicals) and low density polyethylene Mirason™ 11P (available from Mitsui Chemicals) at a weight ratio of 92/8 and 15 parts by weight of hydrogenated aromatic hydrocarbon resin Alcon™ P125 (available from Arakawa Chemical) as the tackifier was melted by an extruder and discharged from the meltblown spinning nozzle. When the mixture was discharged from the nozzle, heated air was blown to the discharged melted material at the outlet of the nozzle, and this meltblown fiber was allowed to stack directly on the spunbonded nonwoven fabric (1) obtained in Production Example 1. As a result, a sheet having a basis weight of 5 g/$m^2$ which comprises the meltblown nonwoven fabric layer and the spunbonded nonwoven fabric laminate was obtained.

Extrusion lamination

An elastomer film having a basis weight of 30 g/$m^2$ was formed by means of a film die (temperature: 220°C) by extruding a thermoplastic polyurethane (Lezamin™ P-211 available from Dainichiseika Color & Chemicals Mfg. Co.; hardness (JISA based on JIS K6301): 81°; viscosity (200°C): 1.0 to 2 x 100 poise; water vapor permeability of the resin: 4,000 g/$m^2$.d as 30 μm thickness film) used as the elastomer (B) having water vapor permeability by use of an extruder (molding temperature pattern: 170°C/200°C/200°C).

On the other hand, the sheet obtained as described above which comprises the meltblown nonwoven fabric layer and the spunbonded nonwoven fabric laminate was fed at 50 m/min., subjected to corona discharge treatment at 30 W/$m^2$, and sent between the nip roll(embossing roll; embossing area ratio: 7%) and the chill roll, so that the aforesaid elastomer film was laminated to the aforesaid sheet comprising the meltblown nonwoven fabric layer and the spunbonded nonwoven fabric laminate. As a result, the nonwoven fabric laminate of the invention was obtained. The roll conditions were such that the temperature was 30°C, the speed was 50 m/min, and the nip linear pressure was 40 kg/cm. Evaluation results of the laminate are shown in Table 10.

Example 14:

A nonwoven fabric laminate was produced by the same method as described in Example 13 except that the basis weight of the film of the thermoplastic elastomer (thermoplastic polyurethane) was 20 g/$m^2$. Evaluation results of the laminate are shown in Table 10.

Comparative Example 6:

An attempt was made to produce a nonwoven fabric laminate by the same method as described in Example 14 except that a nonwoven fabric sheet comprising only a spunbonded nonwoven fabric which has no nonwoven fabric layer as a porous material was used in the lamination with the thermoplastic elastomer film. Evaluation results of the laminate are shown in Table 10.

Example 15:

A film having a basis weight of 20 g/$m^2$ was formed by means of a film die (temperature: 220°C) by extruding a thermoplastic polyester (Hytrel® 3548L available from DuPont; water vapor permeability of the resin: 3,620 g/$m^2$.d as 30 μm thickness film) used as the thermoplastic elastomer by use of an extruder (molding temperature pattern: 190°C/230°C/220°C). On the other hand, the sheet obtained in Example 13 which comprises the meltblown nonwoven fabric layer and the spunbonded nonwoven fabric laminate was fed at 50 m/min., subjected to corona discharge treatment at 30 W/$m^2$, and sent between the nip roll(embossing roll; embossing area ratio: 7%) and the chill roll, so that the sheet was laminated to the elastomer film. As a result, the nonwoven fabric laminate of the invention was obtained. The roll conditions were such that the temperature was 30°C, the speed was 50 m/min, and the nip linear pressure was 40 kg/cm. Evaluation results of the laminate are shown in Table 10.

Example 16:

A nonwoven fabric laminate was produced by the same method as described in Example 15 except that a spunbonded nonwoven fabric (denier of the component fiber: 2.2 d; tensile strength (MD): 510 g/25 mm; tensile strength (CD): 110 g/25 mm) having a basis weight of 18 g/$m^2$ which was produced from a propylene homopolymer having the density of 0.91 g/$cm^2$ and the MFR of 50 g/10 min. (at 230°) was used for the nonwoven fabric. Evaluation results of the laminate are shown in Table 10.

Table 10

| | | Example 13 | Example 14 | Example 15 | Example 16 | Comp. Example 6 |
|---|---|---|---|---|---|---|
| Elastomer film(B) | Material | Polyurethane | Polyurethane | Polyester | Polyester | Polyurethane |
| | Basis weight [g/m$^2$] | 30 | 20 | 20 | 20 | 20 |
| Nonwoven fabric (A) | Material | Propylene/ ethylene copolymer and ethylen/ butene-1 copolymer | Same as left | Same as left | Polypropylene | Propylene/ ethylene copolymer and ethylen/ butene-1 copolymer |
| | Basis weight [g/m$^2$] | 18 | 18 | 18 | 18 | 18 |
| Porous material(c) Basis weight [g/m$^2$] | | Nonwoven fabric 5 | Nonwoven fabric 5 | Nonwoven fabric 5 | Nonwoven fabric 5 | None |
| Interlaminar strength [g/inch] | | 190 | 60 | 100 | 120 | 5 |
| Water vapor permeability [g/ m$^2$ · day] | | 1700 | 2000 | 3000 | 3000 | 3500 |
| Waterproofness [mmAq] | | >2000 | >2000 | >2000 | >2000 | >2000 |

Raw material resin of the nonwoven fabric as the porous material shown in Table 10:

Maleic acid modified(Tafmer A + Mirason 11P) + Alcon P125

Examples 17 to 21:

Evaluation was conducted of the meltblown nonwoven fabrics having the basis weight as shown in Table 11 which were produced by using 19), EEA(1) (Example 17), EVA(1) (Example 18), EMA(1)(Nucrel™ AN-4311 ) (Example19), Ionomer(Himilan™ 1555)(Example 20) and EEA(1) modified with maleic anhydride (Example 21) for the raw material resin of the nonwoven fabric as the porous material used in Example 15. Results of the evaluation are shown in Table 11.

Table 11

| | | Example 17 | Example 18 | Example 19 | Example 20 | Example 21 |
|---|---|---|---|---|---|---|
| Elastomer film (B) | Material | Polyester | Polyester | Polyester | Polyester | Polyester |
| | Basis Weight [g/m$^2$] | 30 | 20 | 20 | 20 | 20 |
| Nonwoven fabric (A) | Material | Propylene/ ethylene copolymer and ethylene/ butene-1 copolymer | Same as left | Same as left | Same as left | Same as left |
| | Basis Weight [g/m$^2$] | 18 | 18 | 18 | 18 | 18 |
| Porous material (C) | | EEA(1) | EVA(1) | EMA (1) | Ionomer | Modified EEA (1) |
| Basis weight [g/m$^2$] | | 5 | 5 | 5 | 5 | 5 |

Table 11 (continued)

| | | Example 17 | Example 18 | Example 19 | Example 20 | Example 21 |
|---|---|---|---|---|---|---|
| Interlaminar strength [g/inch] | | 90 | 70 | 70 | 70 | 110 |
| Water vapor permeability [g/ $m^2$·day] | | 3000 | 3000 | 3000 | 3000 | 3000 |
| Waterproofness [mmAq] | | > 2000 | > 2000 | > 2000 | > 2000 | > 2000 |

Example 22:

An adhesive resin raw material having the specific gravity of 1.6 g/$cm^3$ was obtained by biaxially mixing the filler of calcium carbonate (particle size distribution: 0.5 to 10 μm; average value: 2 μm) with the ethylene/ethylene acrylate copolymer EEA(1) (Evaflex™ EEA-709 available from DuPont-Mitsui Polychemicals; MFR:25 g/10 min(at 190°C)), at a concentration of 60 wt% in weight ratio(ratio between EEA:calcium carbonate) of 40:60). This adhesive resin raw material was extruded by use of an extruder having a diameter of 50 mm at 140°C to 180°C, and film 20 μm in thickness was obtained by use of the T die at 180°C at the speed of 10 m/min. This film was stretched four times (monoaxially in the MD direction) at normal temperature (30°C), and a porous film (apparent density: 1.0 g/$cm^3$) 5 μm in thickness was obtained.

This porous film was heat-embossed together with the nonwoven fabrics (propylene/ethylene and ethylene/1-butene copolymers) obtained in Production Example 1 which have been subjected to corona discharge treatment and have the basis weight of 18 g/$m^2$ at an area ratio of 30% and at 50°C. As a result, the nonwoven fabric and the porous film were laminated.

Next, this laminate was extrusion-laminated with the thermoplastic polyurethane film used in Example 13 by the same as the aforesaid extrusion lamination method, and the nonwoven fabric laminate of the invention was obtained. Results of evaluation of this laminate as conducted in the same manner as the preceding Examples are shown in Table 12.

Example 23:

A nonwoven fabric laminate was produced by the same method as described in Example 22 except that the basis weight of the film of the thermoplastic elastomer (thermoplastic polyurethane) was 20 g/$m^2$.

Evaluation results of the laminate are shown in Table 12.

Example 24:

A nonwoven fabric laminate was produced by the same method as described in Example 23 except that the polyester elastomer Hytrel™ 3548L (water vapor permeability of the resin: 3620 g/$m^2$.d as 30μm thickness film) was used for the thermoplastic elastomer. Evaluation results of the laminate are shown in Table 12.

Table 12

| | | Example 22 | Example 23 | Example 24 |
|---|---|---|---|---|
| Elastomer fiim(B) | Material | Polyurethane | Polyurethane | Polyester |
| | Basis weight [g/$m^2$] | 30 | 20 | 20 |
| Nonwoven fabric (A) | Material | Propylene/ethylene copolymer and ethylen/butene-1 copolymer | Same as left | Same as left |
| | Basis weight [g/$m^2$] | 18 | 18 | 18 |
| Porous material (C) porous film | | EEA(1)/calcium carbonate = 6/4 | Same as left | Same as left |
| Interlaminar strength [g/inch] | | 160 | 100 | 80 |
| Water vapor permeability [g/$m^2$·day] | | 1600 | 2200 | 2700 |
| Waterproofness [mmAq] | | > 2000 | > 2000 | > 2000 |

Example 25:

Manufacture of nonwoven fabric (A)

A stretchable spunbonded nonwoven fabric having the basis weight of 28 g/$m^2$ was obtained by stretching the spunbonded nonwoven fabric(1) produced in Production Example 1 1.3 times in the longitudinal direction by use of a stretching machine in the atmosphere at 130°C. The maximum elongation of this nonwoven fabric in the transverse direction was 220%, and the load required to cause the nonwoven fabric to be stretched 100% was 10 g/25 mm.

Fabrication of the nonwoven fabric layer as a porous material comprising the ethylene polymer containing the polar group

A sheet of the nonwoven fabric laminate whose meltblown nonwoven fabric layer having the basis weight of 10 g/$m^2$ is formed on the spunbonded nonwoven fabric was obtained by causing the meltblown fiber of the ethylene polymer containing the polar group to be stacked directly on the stretchable spunbonded nonwoven fabric obtained by the same method as described in Example 13, using the same polymer as used in Example 13 for the ethylene polymer containing the polar group.

Extrusion lamination

The nonwoven fabric laminate of the invention was obtained by forming an elastomer film having the basis weight of 40 g /$m^2$ by the same method as described in Example 13, using the same thermoplastic polyurethane as used in Example 13 for the thermoplastic elastomer, and laminating this elastomer film with the sheet of the nonwoven fabric laminate obtained as described above. Evaluation results of the laminate are shown in Table 13.

Example 26:

A nonwoven fabric laminate of the invention was produced by the same method as described in Example 17 except that the basis weight of the thermoplastic elastomer film (B) was 20 g/$m^2$. Evaluation results of the laminate are shown in Table 13.

Example 27:

A nonwoven fabric laminate of the invention was produced by the same method as described in Example 14 except that a different grade of thermoplastic polyurethane (Lezamine™ P-210 available from Dainichiseika Color & Chemicals Mfg. Co.; hardness (JISA based on JIS K6301): 92°; viscosity (200°C): 1.5 to 2 x 100 poise) from the thermoplastic polyurethane shown in Example 14 was used for the resin (water vapor permeability of the resin: 4,000 g/$m^2$.d as 30 μm thickness film) of the thermoplastic elastomer film. Evaluation results of the laminate are shown in Table 13. It may be said that in the case of using polyester for the thermoplastic elastomer film, stretching is easier at a low load than in the case of using polyurethane.

Comparative Example 7:

An attempt was made to produce a nonwoven fabric laminate by the same method as described in Example 18 except that the sheet comprising only the spunbonded nonwoven fabric which has no nonwoven fabric layer comprising the ethylene polymer containing the polar group was used in the lamination with the thermoplastic elastomer film. However, the adhesion of the laminate thus obtained was inadequate. Evaluation results of the laminate are shown in Table 13.

Comparative Example 8:

A mixture of SEBS (H1052 available from Asahi Chemical Industry; MFR: 14.5 g/10 min. (here, as determined at a temperature of 230°C and a load of 2.16 kg in accordance with ISO 1139-91); styrene content: 20 wt%) and LLDPE (Neozex™ 23H available from Mitsui Chemicals) at a weight ratio of 80:20 was used for the thermoplastic elastomer. The water vapor permeability of this resin was 100 g/$m^2$.d as 30 μm thickness film. An elastomer film having the basis weight of 30 g/$m^2$ was obtained by extruding the aforesaid resin through a film die (temperature: 220°C) by means of an extruder (molding temperature pattern: 190°C/230°C/220°C). On the other hand, using only the nonwoven fabric obtained in Example 17, this elastomer film (B) was laminated with the nonwoven fabric in the same manner as described in Example 17, and as a result the nonwoven fabric laminate was obtained. Evaluation results of the laminate are shown in Table 13.

Table 13

| | | Example 25 | Example 26 | Example 27 | Comp. Example 7 | Comp. Example 8 |
|---|---|---|---|---|---|---|
| Basis weight [g/m²] | Elastomer film | 40 | 20 | 20 | 20 | 30 |
| | Nonwoven fabric layer (C) | 10 | 10 | 10 | None | None |
| | Nonwoven fabric (A) | 28 | 28 | 28 | 28 | 28 |
| Tensile Properties | Load at 5% elongation [g/25mm] | 80 | 32 | 50 | 42 | 75 |
| | Load at 25% elongation | 140 | 57 | 90 | 75 | 120 |
| | Load at 50% elongation | 220 | 120 | 160 | 138 | 210 |
| | Load at 100% elongation | 270 | 160 | 200 | 172 | 250 |
| | Load at 150% elongation | 350 | 240 | 280 | 258 | 350 |
| | Max. strength [g/25mm] | 500 | 290 | 380 | 303 | 530 |
| | Max. elongation [%] | 200 | 155 | 200 | 120 | 230 |
| Residual Strain | at 50% elongation [%] | 7 | 8 | 7 | 10 | 4 |
| | at 100% elongation [%] | 16 | 15 | 16 | 20 | 9 |
| Interlaminar strength g/2.54cm [g/in.] | | 187 | 50 | 60 | 10 | 220 |
| Water vapor permeability [g/m2.day] | | 2500 | 3500 | 4800 | 1800 | 10 |
| Waterproofness [mm Aq] | | >2000 | >2000 | >2000 | >2000 | >2000 |

## Claims

1. A water-vapour-permeable non-woven fabric laminate showing water vapour permeability of not less than 1,000 $g/m^2$. day and water pressure resistance of not less than 1,000 mm Aq which comprises a layer of non-woven fabric (A) made of an olefin polymer and a layer of elastomer (B) having water vapour permeability which are laminated through a layer of ethylene polymer (C) containing a polar group, selected from ethylene/ethyl acrylate copolymers, ethylene/vinyl acetate copolymers, ethylene/methacrylic acid copolymers, ionomer resins, modified resins obtainable by modifying ethylene/ethyl acrylate copolymers with unsaturated carboxylic acid and modified resins obtainable by modifying low crystalline copolymers of ethylene and an $\alpha$-olefin other than ethylene with unsaturated carboxylic acid.

2. A laminate according to claim 1 wherein the ethylene polymer (C) further comprises at least one additive selected from elastomers having water vapour permeability, tacky resins, inorganic fillers and foaming agents.

3. A laminate according to claim 1 or claim 2 wherein the elastomer (B) is at least one thermoplastic elastomer selected from urethane-based elastomers, polyester-based elastomers and polyamide-based elastomers.

4. A laminate according to any one of claims 1 to 3 wherein the non-woven fabric (A) is a non-woven fabric formed from core-sheath type conjugate fiber or side-by-side-type conjugate fiber.

**5.** A laminate according to any one of claims 1 to 4 wherein the layer of ethylene polymer (C) is porous.

**6.** A laminate according to claim 5 wherein the ethylene polymer (C) is a non-woven fabric or porous film.

**7.** A process which comprises
coextruding a film made of the elastomer (B) and the ethylene polymer (C) onto the non-woven fabric (A) and cooling the coextrudate product by means of a chill roll so as to produce a water-vapour-permeable non-woven fabric laminate according to claim 1.

**8.** A process according to claim 7 wherein the surface of the chill roll has a non-stick coating.

**9.** A process which comprises
forming a layer of meltblown non-woven fabric, made of an ethylene polymer (C), on a spun bonded non-woven fabric (A) and then laminating a film of an elastomer (B), on the side of the meltblown non-woven fabric layer, by extrusion lamination so as to produce a water-vapour-permeable non-woven fabric laminate according to claim 1.

**10.** Use of a non-woven fabric laminate according to any one of claims 1 to 6 in the production of protective covers, packaging materials, jackets, underwear, sanitary goods, disposable diapers or wall paper.

**Patentansprüche**

**1.** Wasserdampfdurchlässiges Vließstofflaminat, welches eine Wasserdampfdurchlässigkeit von nicht weniger als 1000 g/$m^2$ · Tag und eine Wasserdruckresistenz von nicht weniger als 1000 mm Aq aufweist, das einschließt:

eine Vließstoffschicht (A) hergestellt aus einem Olefinpolymer und
eine Schicht eines Elastomers (B) mit Wasserdampfdurchlässigkeit,
welche durch
eine Schicht von Ethylenpolymer (C) laminiert sind, enthaltend eine polare Gruppe,
gewählt aus Ethylen/Ethylacrylatcopolymeren, Ethylen/Vinylacetatcopolymeren, Ethylen/Methacrylsäurecopolymeren, Ionomerharzen, modifizierten Harzen erhältlich durch Modifizierung von Ethylen/Ethylacrylat-copolymeren mit ungesättigten Carbonsäuren und modifizierten Harzen erhältlich durch Modifizierung niedrigkristalliner Copolymere von Ethylen und α-Olefin abgesehen von Ethylen mit ungesättigten Carbonsäuren.

**2.** Laminat gemäß Anspruch 1, worin das Ethylenpolymer (C) außerdem wenigstens ein Additiv, gewählt aus Elastomeren mit Wasserdampfdurchlässigkeit, Klebeharzen, anorganischen Füllmaterialien und Schäummitteln, umfasst.

**3.** Laminat gemäß Anspruch 1 oder Anspruch 2, wobei das Elastomer (B) wenigstens ein thermoplastisches Elastomer, gewählt aus Urethan-basierten Elastomeren, Polyester-basierten Elastomeren und Polyamid-basierten Elastomeren ist.

**4.** Laminat gemäß einem der Ansprüche 1 bis 3, wobei,der Vließstoff ein Vließstoff gebildet aus einer Konjugatfaser vom Kern-Futteral-Typ (core-sheath type) oder eine Konjugatfaser vom Nebeneinander-Typ (side-by-side-type) ist.

**5.** Laminat gemäß einem der Ansprüche 1 bis 4, worin die Schicht des Ethylenpolymers (C) porös ist.

**6.** Laminat gemäß Anspruch 5, worin das Ethylenpolymer (C) ein Vließstoff oder ein poröser Film ist.

**7.** Verfahren, welches umfasst:

Coextrudieren eines Films hergestellt aus dem Elastomer (B) und dem Ethylenpolymer (C) auf den Vließstoff (A) und Kühlen des coextrudierten Produkts mittels einer Kühlwalze um damit ein wasserdampfdurchlässiges Vließstofflaminat gemäß Anspruch 1 herzustellen.

**8.** Verfahren gemäß Anspruch 7, wobei die Oberfläche der Kühlwalze eine nichthaftende Beschichtung hat.

9. Verfahren, welches umfasst:

Ausbilden einer Schicht aus schmelzgeblasenem Vließstoff, hergestellt aus einem Ethylenpolymer (C) auf einem gesponnen-gebundenem (spun bonded) Vließstoff (A) und dann Laminieren eines Films aus einem Elastomer (B) auf der Seite der schmelzgeblasenen Vließstoffschicht, mittels Extrusionslaminierung um damit ein wasserdampfdurchlässigen Vließstofflaminat gemäß Anspruch 1 herzustellen.

10. Verwendung eines Vließstofflaminates gemäß einem der Ansprüche 1 bis 6 bei der Herstellung von schützenden Decken, Verpackungsmaterialien, Jacken, Unterwäsche, Sanitärartikeln, Wegwerfwindeln oder Tapete.

## Revendications

1. Stratifié à tissu non-tissé, perméable à la vapeur d'eau, présentant une perméabilité à la vapeur d'eau non-inférieure à 1000 g/m$^2$ jour et une résistance à la pression de l'eau non-inférieure à 1000 mm d'eau, qui comprend une couche d'un tissu non-tissé (A) en polymère d'oléfine et une couche d'élastomère (B) perméable à la vapeur d'eau, qui sont stratifiées par l'intermédiaire d'une couche de polymère d'éthylène (C) contenant un groupe polaire, choisi parmi les copolymères d'éthylène et d'acrylate d'éthyle, les copolymères d'éthylène et d'acétate de vinyle, les copolymères d'éthylène et d'acide méthacrylique, les résines ionomères, les résines modifiées que l'on peut obtenir en modifiant des copolymères d'éthylène et d'acrylate d'éthyle avec un acide carboxylique insaturé, et les résines modifiées que l'on peut obtenir en modifiant des copolymères faiblement cristallins d'éthylène et d'une $\alpha$-oléfine autre que l'éthylène avec un acide carboxylique insaturé.

2. Stratifié selon la revendication 1, dont le polymère d'éthylène (C) comprend en outre au moins un additif choisi parmi des élastomères perméables à la vapeur d'eau, des résines collantes, des charges minérales et des agents de formation de mousse.

3. Stratifié selon la revendication 1 ou 2, dont l'élastomère (B) est constitué d'au moins un élastomère thermoplastique, choisi parmi les élastomères à base d'uréthane, les élastomères à base de polyester et les élastomères à base de polyamide.

4. Stratifié selon l'une quelconque des revendications 1 à 3, dont le tissu non-tissé (A) est un tissu non-tissé, formé à partir de fibres conjuguées du type coeur-enveloppe ou de fibres conjuguées du type côte-à-côte.

5. Stratifié selon l'une quelconque des revendications 1 à 4, dont la couche de polymère d'éthylène (C) est poreuse.

6. Stratifié selon la revendication 5, dont le polymère d'éthylène (C) est un tissu non-tissé ou un film poreux.

7. Procédé qui comprend la coextrusion d'un film constitué de l'élastomère (B) et du polymère d'éthylène (C) sur le tissu non-tissé (A) et le refroidissement du produit coextrudé au moyen d'un rouleau refroidisseur, de façon à produire un stratifié à tissu non-tissé, perméable à la vapeur d'eau, selon la revendication 1.

8. Procédé selon la revendication 7, dans lequel la surface du rouleau refroidisseur porte un revêtement non-collant.

9. Procédé qui comprend la formation d'une couche de tissu non-tissé, obtenu par soufflage à l'état fondu, constitué d'un polymère d'éthylène (C), sur un tissu non-tissé (A), lié par filage, puis la stratification d'un film d'un élastomère (B) du côté de la couche de tissu non-tissé, obtenu par soufflage à l'état fondu, par stratification par extrusion, de façon à produire un stratifié à tissu non-tissé, perméable à la vapeur d'eau, selon la revendication 1.

10. Utilisation d'un stratifié à tissu non-tissé selon l'une quelconque des revendications 1 à 6, dans la production de couvertures protectrices, de matériaux d'emballage, d'enveloppes, de sous-vêtements, d'articles sanitaires, de couches jetables ou de papier à tapisser.

Fig. 1

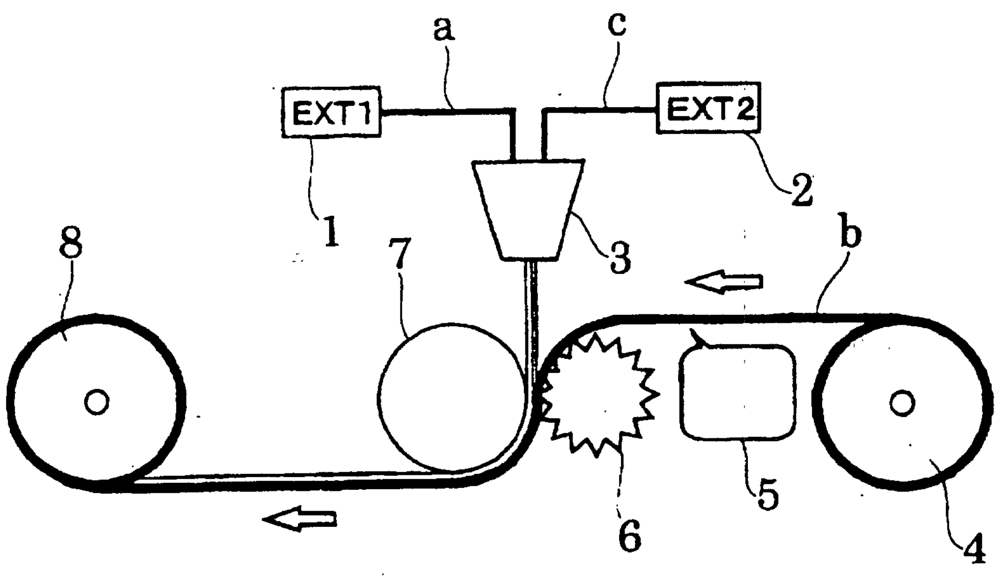


Fig.2

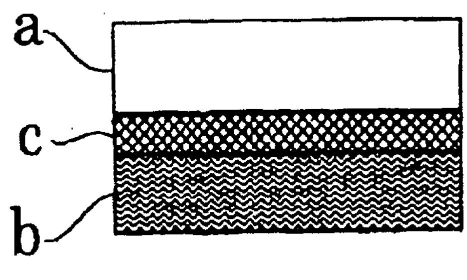

Fig. 3

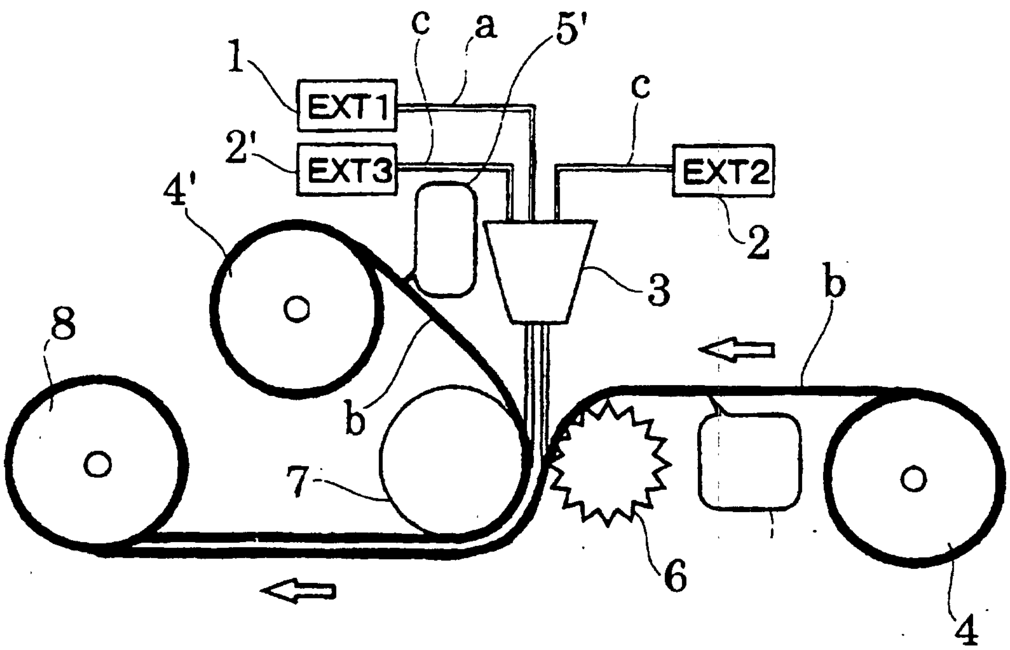


Fig. 4